# EUROPEAN PATENT APPLICATION

(11) **EP 1 532 935 A1**
(43) Date of publication of application: **25.05.2005**
(21) Application number: 04255025.1
(22) Date of filing: 20.08.2004
(51) Int. Cl.: A61B 18/18, A61B 18/14

(54) **Electrosurgical electrode for treating tissue**

(30) Priority: 19.11.2003 US 716714
(71) Applicant: Garito, Jon C., Oceanside, New York 11572 (US); Ellman, Alan G., Oceanside, New York 11572 (US)
(72) Inventor: Garito, Jon C., Oceanside, New York 11572 (US); Ellman, Alan G., Oceanside, New York 11572 (US)
(74) Representative: Cloughley, Peter Andrew

(57) **Abstract**

A bipolar electrode for use with an electrosurgical handpiece, the electrode being configured for use in MIS and other electrosurgical procedures, primarily for endoscopic spinal surgery. The active electrodes use the bipolar principle and are configured to provide more controlled distribution of the electrosurgical currents to the tissue to be modulated. In one embodiment, the bipolar electrodes are formed along a side portion of a longitudinally-extendable tube, each connected to a terminal of the bipolar source. As a result of the bipolar action, the electrosurgical discharge occurs primarily between the adjacent edges of the side-by-side electrodes, which can be described as side-firing or side-emitting bipolar electrodes. A main advantage is that it provides the surgeon with additional control over where exactly the effects of the electrosurgical currents will be obtained. Preferably, the electrodes are mounted on an extendable tube of memory plastic that will assume a desired curved shape when extended to provide more freedom for location of the electrodes by the surgeon to treat tissue during a surgical procedure.

## Description

### RELATED APPLICATION/PATENTS

US application, Ser. No. 09/303,839, filed 5/3/99, commonly owned, for "Electrosurgical Handpiece For Treating Tissue", now Patent No. 6,231,571.

US application, Ser. No. 09/393,286, filed 9/10/99, commonly owned, for "Electrosurgical Handpiece For Treating Tissue", now Patent No. 6,210,409.

US application, Ser. No. 09/483,994, filed 01/18/00, commonly owned, for "Electrosurgical Handpiece For Treating Tissue", now Patent No. 6,352,533.

Published European Patent Application, EP 1 050 279 A1.

This invention relates to a novel electrode construction for use with an electrosurgical handpiece.

### BACKGROUND OF THE INVENTION

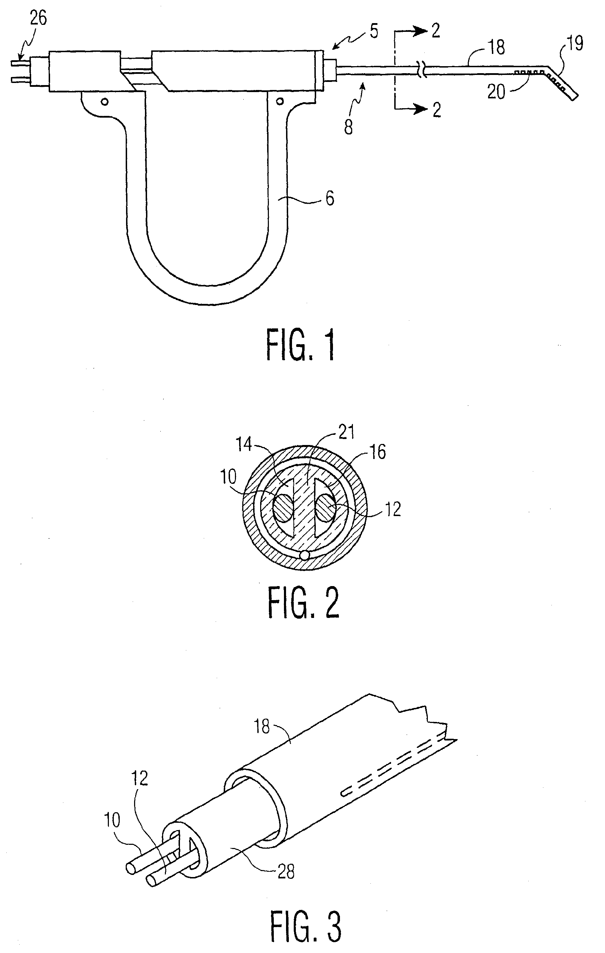

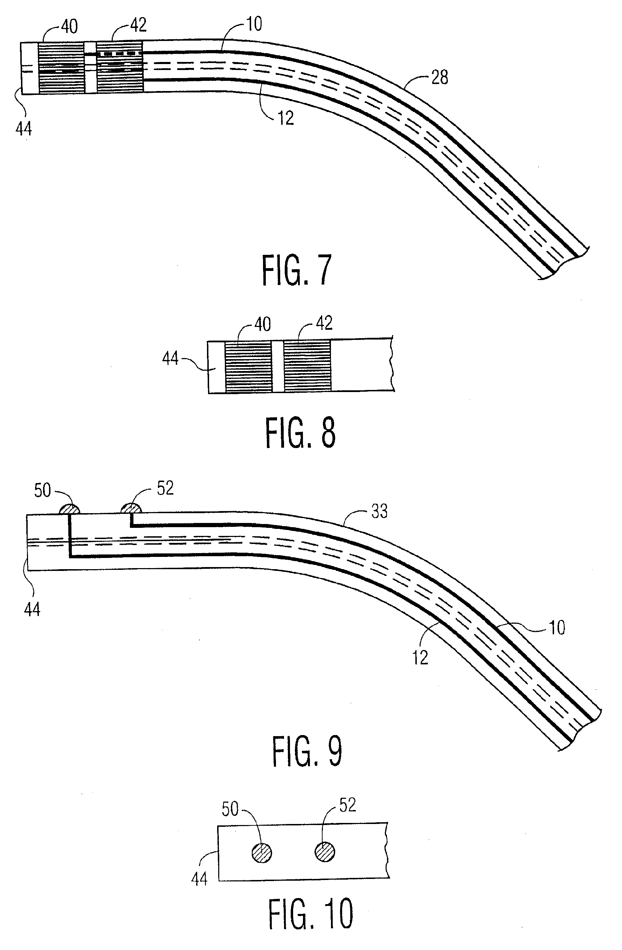

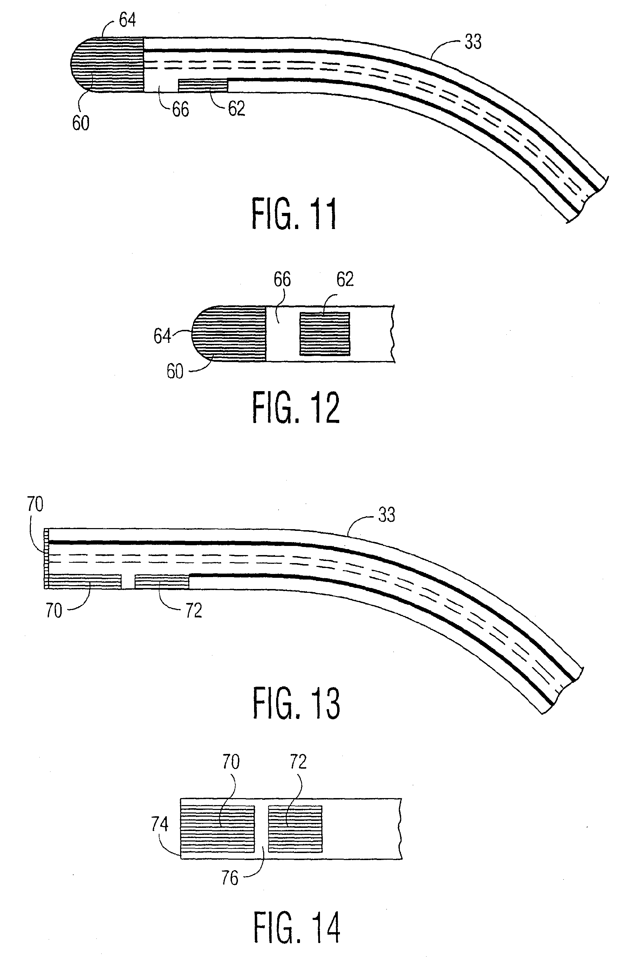

Our prior application, Ser. No. 09/303,839, describes a novel electrosurgical handpiece for treating tissue in a surgical procedure commonly known as minimally invasive surgery (MIS). Among the features described and claimed in the prior application is an electrosurgical handpiece that can be used in MIS and reduces the danger of excessive heat causing possible patient harm. This is achieved in one embodiment by an electrosurgical handpiece that is bipolar in operation and that is configured for use in MIS. The bipolar operation confines the electrosurgical currents to a small active region between the active ends of the bipolar electrode and thus reduces the possibility that excessive heat will be developed that can damage patient tissue. Moreover, the position of the active region can be controlled to avoid patient tissue that may be more sensitive to excessive heat. Preferably, the handpiece is provided with a dual compartment insulated elongated tube, each of the compartments serving to house one of the two wires of the bipolar electrodes. The electrode for MIS use is preferably constructed with a flexible end controllable by the surgeon so as to allow the surgeon to manipulate the end as desired during the surgical procedure. In a preferred embodiment, the flexible end is achieved by weakening at the end the housing for the electrode, and providing a pull string or wire or equivalent mechanism connected to the weakened housing end and with a mechanism at the opposite end for the surgeon to pull the string or wire to flex the housing end to the desired position. This feature allows the surgeon to position the active electrode end at the optimum location for treating, say, a herniated disk to remove undesired regions and to provide controlled heat to shrink the tissue during surgery. In Figs. 3-7 of the prior application, a suitable bipolar electrode is described, which comprises a pair of adjacent rounded electrodes with spaced flat sides separated by an insulating layer. Figs. 8-10 illustrate a suitable unipolar electrode construction of the flexible end handpiece. Fig. 12 illustrates how such an electrode can be used for the reduction of herniated disks in a laparoscopic procedure. The other referenced applications describe variations of these basic constructions, including the use of a plastic for housing the electrode wires and that has memory properties to retain a pre-bent shape, so that when the pulling force is released, the electrode end is restored to its original pre-bent or straight position.

### SUMMARY OF THE INVENTION

The present invention is an improvement of the constructions described and illustrated in the referenced prior patents and publication and hereby incorporates by reference the total contents of U.S. Patents, Nos. 6,352,533, 6,210,409, and 6,231,571, and EP 1 050 279 A1. The present invention describes and claims among other things novel bipolar electrodes for use with a flexible end handpiece. Since the present application otherwise makes use of the same teachings of the prior patents and publication, it was felt unnecessary to repeat in the body of this specification many of the details present in the contents of the prior patents and publication. The present description will be confined solely to the modifications in the electrodes which will still achieve the same or improved benefits as with the constructions of the prior patents and publication. For more details, the reader is directed to the prior patents and publication.

The new electrode constructions of the present improvement uses the bipolar principle and are configured to provide more controlled distribution of the electrosurgical currents to the tissue to be modulated.

In a preferred embodiment, the bipolar electrodes are formed along a side portion of a longitudinally-extendable tube, each connected to a terminal of the bipolar source. As a result of the bipolar action, the electrosurgical discharge occurs primarily between the adjacent edges of the side-by-side electrodes, which will be referred to from time to time as side-firing or side-emitting bipolar electrodes. The main advantage is that it provides the surgeon with additional control over where exactly the effects of the electrosurgical currents will be focused. In typical endoscopic spinal procedures, it is important for the surgeon to be able to access all intra and extra discal soft tissue pathology to ensure a complete procedure is accomplished. As in the earlier referenced patents and publication, a construction is described to provide multiple electrodes of different geometries each of which can be selectively plugged as desired into a common handle or handpiece. In the present invention, each of the electrodes can have different orientations of the side-emitting electrodes, allowing the surgeon to select and use bipolar electrodes having the desired orientation with respect to the tissue being treated and depending upon the orientation of the inserted working channel.

In a first preferred embodiment, the extendable tube is formed of pre-bent nonconductive material with the advantage that the bipolar electrodes can be placed so that when extended outwardly, the electrodes are positioned on the inside or on the outside of the bent end, with the surgeon choosing that electrode whose active electrodes are positioned in the optimal position for the procedure to be conducted.

The constructions of the invention will provide the same important benefits not only for minimally invasive surgery (MIS) of herniated disks but also for other MIS procedures where controlled electrode position and/or controlled heat generation is of importance as described in the prior applications, as well as for general electrosurgical procedures where the volumetric reduction of tissue is desirable.

The various features of novelty which characterize the invention are pointed out with particularity in the claims annexed to and forming a part of this disclosure. For a better understanding of the invention, its operating advantages and specific objects attained by its use, reference should be had to the accompanying drawings and descriptive matter in which there are illustrated and described the preferred embodiments of the invention, like reference numerals designating the same or similar elements.

### BRIEF DESCRIPTION OF THE DRAWINGS

In the drawings:
Fig. 1 is a perspective view similar to that of Fig. 1 of USP 6,231,571 of one form of an electrosurgical handpiece according to the prior patent fitted with a bipolar activator to provide background for understanding the present invention, the electrode being illustrated with the handle in its closed position with the not shown inner electrode tube inside a bent outer tube;
Fig. 2 is a cross sectional view of the handpiece of Fig. 2 along the line 2-2;
Fig. 3 is a partial perspective view of the handpiece of Fig. 1 showing the dual lumens and dual connecting wires;
Fig. 4 is a side view of the working end of the extended electrode tube of an electrosurgical handpiece similar to that of Figs. 16 and 17 of USP 6,231,571, illustrating a preferred embodiment of the present invention with side-emitting electrodes shown in the electrode's extended flexed position;
Fig. 5 is a side view of the embodiment of Fig. 4;
Figs. 6, 7 and 8 are side and perspective views similar to that of Figs. 4 and 5 of a variant;
Figs. 9 and 10 are side views similar to that of Figs. 7 and 8 of a further variant;
Figs. 11 and 12 are side views similar to that of Figs. 7 and 8 of still a further variant;
Figs. 13 and 14 are side views similar to that of Figs. 7 and 8 of still a further variant;
Fig. 15 is a perspective view of the embodiment of Figs. 13 and 14;
Fig. 16 is a side view of still another variant.

### DETAILED DESCRIPTION OF THE PREFERRED EMBODIMENTS

The reader is directed to the referenced prior patents and publication for a more detailed description of the background of the present invention which will assist in understanding the improvements offered by the present application.

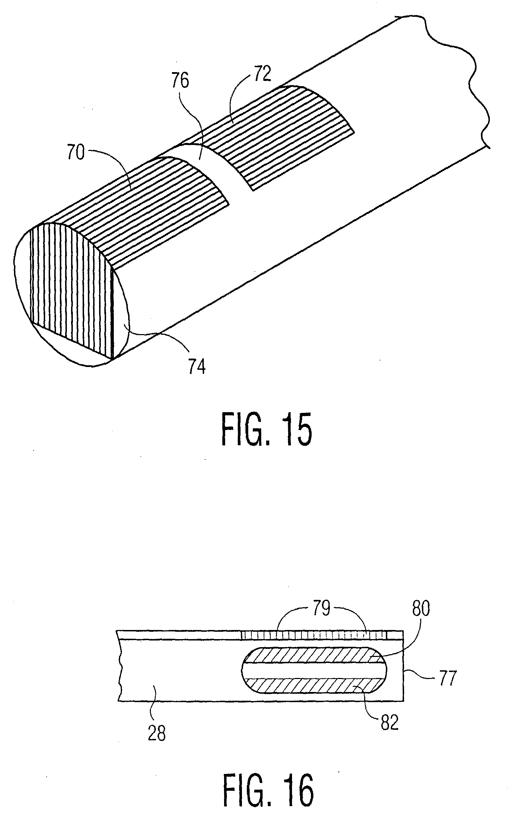

In the present application, the gun configuration 5 remains essentially the same. The present embodiments involve the configuration of the extended electrodes when the handle 6 is squeezed, an internal spring or that of the handle automatically retracting the extended electrodes when the handle is released. The gun is similar to that illustrated in Figs. 16 and 17 of USP 6,231,571. The only changes made are the construction of the active bipolar electrodes at the extended flexible end. For purposes of better understanding the invention, a brief description is given of the gun of Fig. 1 of USP 6,231,571, though it will be clear from the description given below of the embodiments of the present invention that the gun is similar to that illustrated in Figs. 16 and 17 of USP 6,231,571. As in the earlier applications, for the bipolar handpiece shown in Fig. 1, two electrically-insulated wires 10, 12 are passed through insulated compartments, referred to from time-to-time as dual lumens 14, 16, of a tubular housing 18 whose distal end 19 is weakened as by spaced slots 20 and thereby made flexible. The mechanism for bending the end of or for extending the active electrodes is not shown but is the same as or similar to that of the referenced prior patents and publication. The two wires 10, 12 are not only insulated from each other so that bipolar electrosurgical voltages can be applied between them, but they are also insulated from the outer tubular housing 18 which may be of metal or stiff plastic. The latter insulation may be in the form of an inner flexible plastic tube 28.

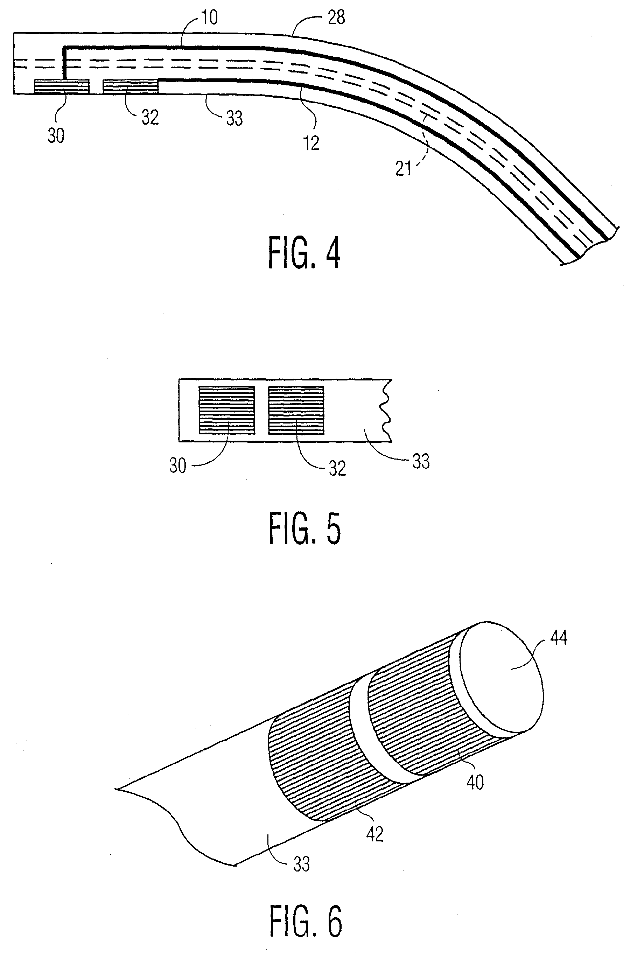

A first preferred embodiment of the present invention is illustrated in Figs. 4 and 5. The two wires 10, 12 which preferably are of round fine wire terminate at the distal end in a pair of side-by-side exposed, bare, i.e., not insulated, longitudinally-spaced conductive, for example, of metal, area electrodes 30, 32 on the outside side wall 33 of the inner flexible tube 28 and which serve as the active bipolar electrodes of the working end and which longitudinally projects from the end of the outer stiff tube 18 (not shown in Figs. 4 and 5) when the handle is squeezed. The inner tube 28 adopts a curved configuration by reason of the use of pre-bent memory plastic for the inner tube 28 when extended. The dashed lines in the center can represent the separating wall 21 described in the earlier patents. It can also represent an irrigation duct for carrying irrigating fluids to the surgical site as described in the prior patents and publication.

By "longitudinal" is meant generally in the same direction as the long axis of the outer tube 18. By "lateral" is meant transverse to that long axis. In Fig. 4, the area electrodes 30, 32, which as an example may have an area of about 3-4 mm² , are shown in their normal extended working position on the inside of the curved concave extendable part. They of course can be provided on the opposite convex side, or preferably a family of electrodes is constituted of several electrode assemblies with the electrodes on various sides of the extendable part as well as straight electrodes. The two insulated wires 10, 12 terminate at the left end of the gun 5 in a connector 26 (Fig. 1) having prongs which allows the gun to be plugged into the standard bipolar cable which connects the gun to conventional electrosurgical apparatus. If a pulling wire (not shown) is needed, it may be anchored as by welding to the flexible end at, for example, a bottom point (when the bending is to occur downward). The electrodes 8 themselves can be removed from the gun and replaced by other electrodes in the same family. When the surgeon while holding the gun handle 6 squeezes it, the inner mechanism is activated pushing the inner tube 28 end out of the outer housing 18 as described in the prior patents and causing the end to curve if pre-bent. The surgeon can manipulate the position of the active electrodes 30, 32 by the force exerted on the handle or whatever other mechanism is employed to cause the inner tube to extend from the end.

The position of the extended part can be oriented in various planes by use of the memory material to control the plane of flexing.

Once the surgeon has positioned the working end of the handpiece with respect to the tissue to be operated on, he or she then activates the electrosurgical apparatus causing a discharge of electrosurgical bipolar currents between the bare electrode regions 30, 32 capable of causing excision or shrinkage of tissue or cauterization of a blood vessel in the usual way positioned adjacent the exposed electrodes. Other usable mechanical or electrical structures following the teachings of the prior applications will be appreciated by those skilled in this art. As with the embodiments of the prior patents, the insulating tube 28 will prevent accidental conductive touching of patient tissue, so that the bipolar discharge is localized to the spacing between the bare regions 30, 32. When the pressure on the handle is released, the extended tube end 28 automatically retracts inside the outer housing 18.

In the patented embodiments, the tubular housing 18 can be of relatively stiff metal that will not bend except where desired at the area of the slots 20 when provided. For example, a suitable metal is stainless steel and a suitable tube wall thickness is about 0.002-0.01 inches. The tube outside diameter is typically about 0.04-0.1 inches. The two exposed electrode regions 30, 32 can be spaced apart in its plane about 1-2 mm. The insulation between the two wires 30, 32 can be provided, for example, by internal glue, a plastic tube, or a heat-shrunk tube. Other electrically-insulating materials can be substituted. For the application of shrinking herniated tissue via a cannula, the tubular housing is typically about 15-20 inches long.

In the first embodiment of Figs. 4 and 5, the two electrode regions 30, 32 are configured as squares, but they can have other shapes as well.

In the second embodiment of Figs. 6-8, the two electrodes 40, 42 completely encircle its supporting insulating inner tube 33. As in the first embodiment, the electrodes 40, 42 are offset longitudinally from one another and thus extend different distances from the free end 44 of the insulating tubes 33. As shown in Fig. 7, the distal electrode 40 is connected to the wire 10, and the proximal electrode 42 is connected to the wire 12.

In the third embodiment of Figs. 9-10, the two electrodes 50, 52 are in the form of nail or pin heads jutting out laterally from their supporting insulating inner tube 33. In the first embodiment, the electrodes 30, 32 are preferably embedded in the surface of the supporting tube 33. In the second embodiment, the electrodes 40, 42 can be plated or adhered to the surface of the supporting tube 33. In the third embodiment, the electrodes 50, 52 can be anchored to the ends of the connecting wires 10, 12 and thus supported in position at the side of its supporting tube 33.

In the fourth embodiment of Figs. 11 and 12, of the two electrodes 60, 62, the end one 60 covers the entire end 64 of its supporting insulating inner tube 33, which end 64 is rounded. The electrosurgical currents emanate from the region 66 between the adjacent edges of the side emitting electrode regions 60, 62.

In the fifth embodiment of Figs. 13-15, the electrode configurations 70, 72 are similar to that of Figs. 11 and 12, except that the end 74 is flat and the end electrode 70 covers only part of its supporting insulating inner tube 33. The electrosurgical currents emanate mainly from the region 76 between the adjacent edges of the side-emitting electrode regions 70, 72.

Preferably, the electrosurgical currents are RF currents at frequencies exceeding 1.4 MHz, 3.8-4 MHz being preferred.

As mentioned, an irrigation duct can be included inside the inner tube 28 so as to be extendable with the latter, the electrode end free of the electrodes being provided with holes positioned such that the irrigating fluids are expelled near the surgical site receiving the electrosurgical currents. Fig. 16 illustrates this embodiment, with irrigation holes 79 located at the end 77 of the extendable tube 28. While the embodiments with longitudinally-spaced electrodes are preferred, it is also possible to mount the two electrodes 80, 82 side-by-side spaced laterally from one another, circumferentially as shown in Fig. 16, but preferably extending only over a small circumferential area so that the electrosurgical currents can be concentrated at a localized area or site adjacent where the two electrodes 80, 82 are placed.

Instead of memory plastic for the extendable tube 28, a pre-stressed helical spring or spring wire can be mounted in the inner tube such that when the inner tube 28 is extended, the electrode end assumes a desired curvature. Retracting such an inner tube will cause the latter to straighten while within the outer housing 18.

In the embodiments illustrated, the flexible end with the side-emitting electrodes is shown curving CCW, but it will be understood that it can easily be arranged to curve CW and thus in the opposite direction, or even made straight if desired.

While stiff plastic can be used, the preferred embodiments use a metal for the outer tubular housing 18. With a bipolar assembly, the electrode wires have to be insulated from each other and from the metal tube. The 2-compartment electrically-insulating liner tube 28 serves this function. However, in principle, if the electrical connecting wires have their own good electrically-insulating coating, then the insulating liner can be dispensed with.

During operation of the handpiece according to the invention, the electrosurgical currents are concentrated between the side edges of the bare exposed conductive regions 30, 32 across which the active voltage is developed. The electrode is chosen by the surgeon for obtaining the best results during the excision of tissue or blood vessel coagulation.

It will also be understood that, while the invention has been described with reference to a gun having a stiff outer tube, the latter can have some flexibility, so long as it is stiffer than the inner tube. This can be an advantage if the outer tube has an end that can be pre-bent at the manufacturer's end or by the surgeon into a fixed position (as shown in Fig. 1) and then, when the handle is squeezed, the inner tube with the electrodes will be extended in the direction of the pre-bent end of the outer tube. This thus gives an extra degree of freedom to the surgeon's control of the location of the active electrodes at the surgical site.

It will also be understood that, while the invention has been described for use at frequencies higher than 1.4 MHz, this is actually a preferred range, and it can also be used at lower frequencies in the KHz range.

While the invention has been described in connection with preferred embodiments, it will be understood that modifications thereof within the principles outlined above will be evident to those skilled in the art and thus the invention is not limited to the preferred embodiments but is intended to encompass such modifications.

## Claims

1. An electrosurgical electrode assembly for use in an electrosurgical handpiece comprising:
(a) an elongated tubular first member having a first end and a distal flexible extendable second end and a longitudinal axis,
(b) first and second electrically-conductive wires positioned in electrically-insulating relationship in the first member with first means connected to the first member at its first end for applying to the first and second wires a bipolar electrosurgical voltage capable of transmitting electrosurgical currents along the wires,
(c) first and second spaced exposed electrodes mounted on the side of the first member at its extendable second end and being connected respectively to the first and second wires,
(d) wherein electrosurgical currents are generated sideways between the first and second electrodes when the electrosurgical voltage is applied to the first and second wires.

2. The electrosurgical electrode assembly as claimed in claim 1, wherein the spaced first and second electrodes are spaced apart longitudinally.

3. The electrosurgical electrode assembly as claimed in claim 2, wherein the first and second electrodes occupy only a small circumferential region of the tubular first member.

4. The electrosurgical electrode assembly as claimed in claim 2, wherein the first and second electrodes encircle circumferentially the tubular first member.

5. The electrosurgical electrode assembly as claimed in claim 2, wherein the first and second electrodes are nail heads protruding from the side of the tubular first member.

6. The electrosurgical electrode assembly as claimed in claim 2, wherein the first electrode covers substantially an end surface of the tubular first member.

7. The electrosurgical electrode assembly as claimed in claim 6, wherein the first electrode covers substantially the whole end of the tubular first member.

8. The electrosurgical electrode assembly as claimed in claim 1, wherein the first and second electrodes are circumferentially spaced.

9. An electrosurgical electrode assembly as claimed in claim 1, in combination with the electrosurgical handpiece wherein:
I) the handpiece comprises a gun-shaped member having a handle,
II) the electrode assembly being mounted in the handle such that, when the handle is squeezed, the tubular first member is extended outwardly.

10. The combined electrosurgical electrode assembly and electrosurgical handpiece as claimed in claim 9, further comprising an irrigation duct within the tubular first member, and apertures at the end of the first member for expelling irrigation fluid near the place where the electrosurgical currents are generated.

11. The combined electrosurgical electrode assembly and electrosurgical handpiece as claimed in claim 9, wherein the electrosurgical voltage has a frequency in excess of 1.4 MHz.

12. The combined electrosurgical electrode assembly and electrosurgical handpiece as claimed in claim 11, wherein the electrosurgical voltage has a frequency between 3.8 and 4 MHz.

13. The combined electrosurgical electrode assembly and electrosurgical handpiece as claimed in claim 9, wherein the handpiece comprises an outer relatively stiff tube with a bendable end, the elongated tubular first member being mounted inside the outer tube so as to be extendable and retractable when the handle is squeezed and released, respectively.
